# EUROPÄISCHE PATENTSCHRIFT

(11) **EP 1 356 780 B1**
(45) Veröffentlichungstag und Bekanntmachung des Hinweises auf die Patenterteilung: **14.06.2006**
(21) Anmeldenummer: 03007582.4
(22) Anmeldetag: 02.04.2003
(51) Int. Cl.: A61B 18/14

(54) **Ablationsvorrichtung**
Ablation device
Dispositif d'ablation

(30) Priorität: 24.04.2002 US 128576
(43) Veröffentlichungstag der Anmeldung: 29.10.2003
(73) Patentinhaber: BIOTRONIK GmbH & Co. KG, 12359 Berlin (DE)
(72) Erfinder: Natale, Andrea Dr., OH 44022-1073 (US)
(74) Vertreter: Lindner-Vogt, Karin L.

(56) Entgegenhaltungen:
- WO-A-01/37925
- US-A1- 2002 022 839

## Beschreibung

Die Erfindung betrifft eine Ablationsvorrichtung für Herzgewebe, insbesondere zur Erzeugung einer zirkulären Läsion im Bereich einer Gefäßmündung im Herzen nach dem Oberbegriff des Patentanspruches 1.

Zum Hintergrund der Erfindung ist festzuhalten, dass die Katheterablation eine vermehrt eingesetzte Therapie zur Behandlung bestimmter Arrhythmien ist. Dabei wird an einer bestimmten Stelle im Herzmuskelgewebe mit Hilfe des Ablationsapplikators des Katheters eine Läsion - also eine Gewebedenaturierung in der Art einer Gewebevernarbung - erzeugt, um dort die für die Arrhytmien verantwortlichen, fehlerhaften elektrischen Reizleitungsbahnen zu unterbrechen. Die Energie-Einbringung in das Herzmuskelgewebe über den Ablationsapplikator erfolgt dabei in aller Regel über Ablationselektroden, die mit Hochfrequenzstrom arbeiten. Zur Ablation können ferner andere Energieformen, wie Mikrowellenenergie, Hochspannungsgleichstrom oder prinzipiell andere Denaturierungsmechanismen, wie Kälte oder Chemikalien (z.B. Alkohol), eingesetzt werden. Der Begriff "Ablationsapplikator", wie er in der vorliegenden Anmeldung auch in Verbindung mit dem eigentlichen Erfindungsgegenstand benutzt wird, soll grundsätzlich alle genannten Ablationsmöglichkeiten umfassen, wobei Ablationselektroden die gängigste Variante darstellen.

Aus einer Vielzahl von dem jeweiligen Anwendungszweck angepassten Varianten von Ablationskathetern sei als Stand der Technik die WO 98/49957 A1 herausgegriffen, die eine Ablationsvorrichtung zur Erzeugung linearer Läsionen zwischen den Mündungsöffnungen zweier Pulmonalvenen in den Vorhof des Herzens zeigt. Entsprechend dem Oberbegriff des Patentanspruches 1 ist dabei ein steuerbarer Katheter vorgesehen, der vor seinem distalen Ende eine Fixiervorrichtung in Form eines dilatierbaren Ballons zur Festlegung des Katheters im Ostium der Pulmonalvene trägt.

Bei dieser bekannten Ablationsvorrichtung dient der Katheter nicht nur zur Basispositionierung des Ablationsapplikators, sondern er trägt die entsprechenden Ablationselektroden selbst auf seinem Schaft. In seiner speziellen Ausgestaltung kann nun proximal von den Ablationselektroden der Schaft des Katheters durch eine zweite Führungseinrichtung vor die Mündungsöffnung einer zweiten Pulmonalvene verbracht werden, sodass sich die linear aneinander gereihten Ablationselektroden auf die Verbindungslinie zwischen den beiden Mündungsöffnungen zweier benachbarter Pulmonalvenen legen. Damit ist auf zuverlässige Weise eine lineare Läsion zwischen den beiden Mündungsöffnungen anzubringen.

Weitere Konfigurationen von Ablationskathetern sind beispielsweise der US 5,239,999 A, der WO 95/15115 A1 bzw. der WO 95/31111 A1 entnehmbar. Dort sind Ablationselektroden in unterschiedlich gewendelter oder leicht gebogener Form gezeigt.

Jüngere Untersuchungen haben gezeigt, dass gerade zur Therapie des arterialen Herzflimmerns zirkuläre Läsionen um die oder an den Mündungen der Pulmonalvenen (im Folgenden: PV-Mündung) in das Atrium hinein gute Erfolge erzielen.

Die bekannten Ablationsvorrichtungen sind für derartig geformte Läsionen praktisch nicht geeignet, da eine ringförmige Anlage der Ablationselektroden um oder an der PV-Mündung nicht oder nur sehr schwer realisierbar ist. Die WO 01/37925 A2 zeigt hier zwar einen gewisen Lösungsansalt, indem ein Katheter mit einer widerlagervorrichtung in Form eines Ballons sowie ein zweiter Katheter mit einem kreisartig expandierbaren tionsapplikator vorgeschlagen werden. Diese Zwei-Katheter Anordnung ist allerdings aufwendig ist konstruktion und Applikation. Insofern liegt der Erfindung die Aufgabe zugrunde, eine Ablationsvorrichtung anzugeben, mit der auf zuverlässige und applikationstechnisch einfache Weise eine zirkuläre Läsion um eine oder an einer Gefäßmündung im Herzen erzeugt werden kann.

Diese Aufgabe wird durch die im Kennzeichnungsteil des Anspruches 1 angegebenen Merkmale gelöst. Demnach ist ein Katheter mit einem linearen Ablationsapplikator vorgesehen, der distal vor der Widerlagervorrichtung des Katheters angeordnet und von einer gestreckten Passivstellung in eine radial expandierte, kreisbogenartig umlaufende Ablationsstellung verbringbar und dabei mittels eines Fuhrungshülse zumindest in seinem distalen Endbereich axial gegenüber der Widerlagervorrichtung verschiebbar ist.

Durch die erfindungsgemäße Ausgestaltung des Katheters wird durch den in Ablationsstellung kreisbogenartig umlaufenden Ablationsapplikator dieser quasi forminhärent in eine korrekte Stellung zur Anbringung der zirkulären Läsion verbracht und durch die Widerlagervorrichtung sauber gehalten. Insoweit wird eine hohe Applikationssicherheit unter entsprechender Verbesserung des Therapieerfolges erzielt.

Dabei ist der Ablationsapplikator distal vor der Widerlagervorrichtung angeordnet, sodass an der PV-Mündung eine Läsion gelegt werden kann.

Bei der Widerlagervorrichtung handelt es sich vorzugsweise um einen dilatierbaren Ballon am Schaft des Positionierkatheters. Der Ballondurchmesser in inflatiertem Zustand muss dabei größer als der Durchmesser des betroffenen Gefäßes sein, d. h. er liegt in einer Größenordnung von ca. 15 mm und mehr. Der Ballon wird dabei mit Hilfe des Katheters gegen den Gefäßwandbereich um die PV-Mündung gedrückt und bildet somit ein Widerlager für den distal vor ihm liegenden Ablationsapplikator. Dieser kann damit - wie aus der Beschreibung des Ausführungsbeispiels noch näher deutlich wird - sauber in den "Zwickelbereich" zwischen der Ballon-Frontseite und der PV-Mündung gehalten werden.

Um die Bildung der kreisbogenartig umlaufenden Ablationsstellung zu unterstützen, ist der Ablationsapplikator vorzugsweise durch eine Mehrfach-Elektrodenanordnung gebildet, deren in Axialrichtung aneinandergereihte Einzelelektroden aus einem hochflexiblen Werkstoff- beispielsweise jeweils aus einer Spiralwicklung oder aus flexiblem, leitenden Kunststoffbestehen.

Durch eine Überdeckung von mindestens 180° durch den Ablationsapplikator wird gewährleistet, dass eine komplett umlaufende zirkuläre Läsion mit nur einer Verdrehung des Ablationskatheters erzielbar ist.

Wenngleich dies nicht umittelbar Gegenstand der Erfindung ist, ist darauf hinzuweisen, dass der Katheter mit bekannten Maßnahmen zur Kontrolle der korrekten Lage versehen sein kann. So kann die Lage sonographisch über einen an der Spitze des Katheters angeordneten Ultraschallwandler oder über ein bipolares Elektrogramm kontrolliert werden, das durch eine bipolare Elektrodenanordnung an der Spitze des Katheters abgeleitet werden kann. Auch kann der Katheter über zusätzliche Lumen zur Injektion von Röntgenkontrastmittel verfügen, das über das Lumen in die Pulmonalvene zu deren angiographischer Darstellung injiziert wird.

Weitere Merkmale, Einzelheiten und Vorteile der Erfindung ergeben sich aus der nachfolgenden Beschreibung, in der Ausführungsbeispiele des Erfindungsgegenstandes anhand der beigefügten Zeichnungen näher erläutert werden. Es zeigen:
- Fig. 1: eine schematische Teildarstellung einer Ablationsvorrichtung in Passivstellung in einer ersten Ausführungsform und
- Fig. 2: eine Darstellung analog Fig. 1 in Ablationsstellung der Vorrichtung.

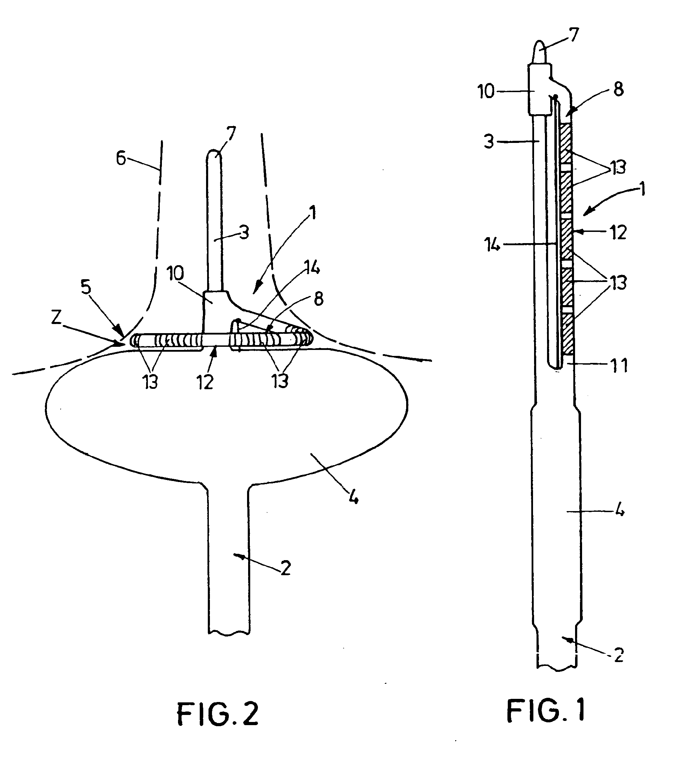

Wie aus Fig. 1 deutlich wird, weist die als Ganzes mit 1 bezeichnete Ablationsvorrichtung einen steuerbaren Katheter 2 auf, der vor seinem distalen Ende 3 mit einem dilatierbaren Ballon 4 versehen ist. In Fig. 1 ist der Ballon 4 in seinem nicht expandierten Ausgangszustand, in Fig. 2 in seinem aufgeweiteten Zustand gezeigt, in dem er vor einer in Fig. 2 strichliert dargestellten Mündungsöffnung 5 einer Pulmonalvene 6 in das Atrium des Herzens liegt. Der Katheter 2 kann übliche Zusatzeinrichtungen aufweisen und beispielsweise mit einem Lumen für einen Führungsdraht, einer Deflektionseinrichtung zur gezielten Steuerung des distalen Endes 3 usw. versehen sein. Weitere Zusatzausrüstungen wurden ferner in der Beschreibungseinleitung bereits genannt.

Distal vor dem Ballon 4 ist der Katheter 2 axial in das distale Steuerende 7 und eine als Ganzes mit 8 bezeichnete Ablationsspitze geteilt. Das freie Ende der Ablationsspitze 8 ist über eine Führungshülse 10 axial verschiebbar auf dem Steuerende 7 des Katheters 2 geführt.

Im Bereich zwischen der Führungshülse 10 und dem Übergang 11 zum eigentlichen Katheter 2 ist an der Ablationsspitze 8 ein Ablationsapplikator 12 in Form von fünf aneinandergereihten Ringelektroden 13 vorgesehen, die jeweils aus hochflexiblem Spiraldraht bestehen. Über diese Ringelektroden 13 kann ein Hochfrequenzstrom an damit in Kontakt gelangendes Gewebe abgegeben werden, um eine Läsion zu erzeugen.

In Fig. 1 ist die gestreckte Lage des Ablationsapplikators 12 dargestellt, aus der er mit Hilfe eines im Katheter 2 vertaufenden Zugdrahtes 14 in die in Fig. 2 gezeigte, radial expandierte Ablationsstellung verbringbar ist. Dazu ist der Zugdraht 14 im Bereich der distalen Führungshülse 10 befestigt und läuft im Bereich des Überganges 11 in den Ablationskatheter 8 ein. Durch Züge am Zugdraht 14 wird die Ablationsspitze 8 das Steuerende 7 des Positionierkatheters 2 entlang in proximaler Richtung verschoben, sodass sich die Ablationsspitze 8 im Bereich des Ablationsapplikators 12 aufweitet und in eine kreisbogenartig umlaufende Konfiguration durch entsprechende Vorformung der Ablationsspitze 8 verbracht wird. Dabei kann der Positionierkatheter 2 mit dem Ballon 4 gegen die Herzwand um den Bereich der Mündungsöffnung 5 gedrückt werden. Dadurch wird eine Art Widerlager geschaffen, gegen das der Ablationsapplikator 12 mit Hilfe des Zugdrahtes 14 gezogen werden kann. Dieser schmiegt sich dadurch innig in den ringförmig umlaufenden Zwickelbereich Z ein, der durch die auslaufende Mündungsöffnung 5 und die Vorderseite des Ballons 4 gebildet wird. Der Ablationsapplikator 12 legt sich damit eng an das zu ablatierende Gewebe an, sodass die Gewebedenaturierung mit einem hohen Wirkungsgrad erfolgt. Der Ablationsapplikator 12 überdeckt dabei ferner einen Peripherwinkel von mehr als 180°, sodass sich in dieser der Ablationsapplikator 12 über mehr als die Hälfte des Umfangs der zu erzeugenden zirkulären Läsion erstreckt.

Im Folgenden soll kurz die Erzeugung der zirkulären Läsion anhand der Fig. 1 und 2 dargestellt werden. So wird der Positionierkatheter 2 bei nicht dilatiertem Ballon 4 über eine transseptale Punktion in den linken Vorhof des Herzens eingebracht, wo mit üblichen Mitteln die Einmündungen aller Pulmonalvenen sondiert werden. Nach Bestätigung der korrekten Lage des distalen Endes 3 des Positionierkatheters 2 in der Mündungsöffnung 5 der gewünschten Pulmonalvene 6 wird der Ballon 4 dilatiert und gegen die Herzwand um die Mündungsöffnung 5 gedrückt.

Anschließend wird die Ablationsspitze 8 auf dem Steuerende 7 des Positionierkatheters 2 in proximaler Richtung geschoben, bis die in Fig. 2 dargestellte Position vor dem Ballon 4 des Positionierkatheters 2 erreicht ist. Der Ablationsapplikator 12 liegt also distal vom Ballon 4 in der Mündungsöffnung 5. In dieser Position liegen die Ringelektroden 13 über einen Peripherwinkel an der Gefäßwand an. Durch Abgabe eines hochfrequenten Stromes wird ein Teil der zirkulären Läsion erzielt. Anschließend wird der Ablationsapplikator 12 zumindest teilweise in die in Fig. 1 gezeigte Passivstellung verbracht, um ca. 180° gedreht und wieder kreisbogenartig in die in Fig. 2 gezeigte Ablationsstellung aufgeweitet. Damit liegt der Ablationsapplikator 12 in dem Bereich an der PV-Mündung an, der vorher noch nicht mit einer Läsion versehen worden ist. Durch eine nochmalige Stromabgabe wird die zirkuläre Läsion geschlossen.

## Patentansprüche

1. Ablationsvorrichtung für Herzgewebe, insbesondere zur Erzeugung einer zirkulären Läsion in einer Gefäßmündung (5) im Herzen, umfassend
- einen Katheter (2), der im Bereich seines distalen Endes (3) mit einer Widerlagervorrichtung (4) zum Halten des distalen Endes (3) des Katheters (2) an einer kardialen Gefäßmündung (5) versehen ist,
- einen linearen Ablationsapplikator (12), der distal vor der Widerlagervorrichtung (4) des Katheters (2) angeordnet und von einer gestreckten Passivstellung in eine radial expandierte, etwa kreisbogenartig umlaufende Ablationsstellung verbringbar ist, der Ablationsapplikator (12) zumindest in seinem distalen Endbereich mittels einer Führungshülse (10) verschiebbar auf dem distalen Endabschnitt (3) des Katheters (2) geführt ist, wobei durch axiales Verschieben der Führungshülse (10) in proximaler Richtung der Ablationsapplikator (12) in die Ablationsstellung verbringbar ist, **dadurch gekennzeichnet, dass**

2. Ablationsvorrichtung nach Anspruch 1, **dadurch gekennzeichnet, dass** der Ablationsapplikator (12) axial gegenüber der Widerlagervorrichtung (4) verschiebbar ist.

3. Ablationsvorrichtung nach einem der vorgenannten Ansprüche, **dadurch gekennzeichnet, dass** die Widerlagervorrichtung durch einen dilatierbaren Ballon (4) am Schaft (7) des Katheters (2) gebildet ist.

4. Ablationsvorrichtung nach einem der vorgenannten Ansprüche, **dadurch gekennzeichnet, dass** die Steuerung des Ablationsapplikators (12) von der Passiv- in die Ablationsstellung durch eine Zugdrahtkinematik (14) vornehmbar ist.

5. Ablationsvorrichtung nach einem der vorgenannten Ansprüche, **dadurch gekennzeichnet, dass** der Ablationsapplikator (12) durch eine Mehrfach-Elektrodenanordnung gebildet ist, deren in Axialrichtung aneinandergereihte Einzelelektroden (13) aus einem hochflexiblen Werkstoff bestehen.

6. Ablationsvorrichtung nach einem der vorgenannten Ansprüche, **dadurch gekennzeichnet, dass** der Ablationsapplikator (12) in seiner kreisbogenartig umlaufenden Ablationsstellung einen Peripherwinkel (P) von mindestens 180° überdeckt.

## Claims

1. An ablation device for cardiac tissue, particularly for generating a circular lesion in a vessel mouth (5) in the heart, comprising
- a catheter (2), which is provided in the area of its distal end (3) with a buttress device (4) for holding the distal end (3) of the catheter (2) on a cardiac vessel mouth (5),
- a linear ablation applicator (12), which is positioned distally in front of the buttress device (4) of the catheter (2) and may be brought from a stretched passive position into a radially expanded ablation position having a periphery approximately in the shape of a circular arc,
**characterized in that** the ablation applicator (12) is guided displaceably on the distal end section (3) of the catheter (2) at least in its distal end area using a guide sleeve (10), the ablation applicator (12) being able to be brought into the ablation position through axial displacement of the guide sleeve (10) in the proximal direction.

2. The ablation device according to Claim 1, **characterized in that** the ablation applicator (12) is axially displaceable in relation to the buttress device (4).

3. The ablation device according to one of the preceding claims, **characterized in that** the buttress device is formed by a dilatable balloon (4) on the shaft (7) of the catheter (2).

4. The ablation device according to one of the preceding claims, **characterized in that** the control of the ablation applicator (12) from the passive position into the ablation position may be performed by a tension wire kinematic (14).

5. The ablation device according to one of the preceding claims, **characterized in that** the ablation applicator (12) is formed by a multiple electrode arrangement, whose individual electrodes (13), which are arrayed in the axial direction, are made of a highly flexible material.

6. The ablation device according to one of the preceding claims, **characterized in that** the ablation applicator (12) covers a peripheral angle (P) of at least 180° in its ablation position having a periphery in the shape of a circular arc.

## Revendications

1. Dispositif d'ablation pour tissu cardiaque, notamment pour la création d'une lésion circulaire dans une embouchure de vaisseau (5), dans le coeur, comprenant :
- un cathéter (2) qui est équipé au niveau de son extrémité distale (3) d'un dispositif de butée (4) pour maintenir l'extrémité distale (3) du cathéter (2) sur une embouchure de vaisseau du coeur (5),
- un applicateur d'ablation linéaire (12) qui est disposé en amont au niveau distal du dispositif de butée (4) du cathéter (2) et peut être amené d'une position passive étirée à une position d'ablation en expansion radiale et approximativement périphérique en forme d'arc de cercle, **caractérisé en ce que**
le cathéter d'ablation (12), du moins dans sa zone terminale distale, est guidé au moyen d'un manchon de guidage (10) de manière mobile sur la section terminale distale (3) du cathéter (2), tandis que, par déplacement axial du manchon de guidage (10) dans le sens proximal, l'applicateur d'ablation (12) peut être amené dans la position d'ablation.

2. Dispositif d'ablation selon la revendication 1, **caractérisé en ce que** l'applicateur d'ablation (12) est mobile axialement par rapport au dispositif de butée (4).

3. Dispositif d'ablation selon une des revendications précédentes, **caractérisé en ce que** le dispositif de butée est constitué par un ballon dilatable (4) placé sur la tige (7) du cathéter (2).

4. Dispositif d'ablation selon une des revendications précédentes, **caractérisé en ce que** le contrôle de l'applicateur d'ablation (12) depuis la position passive vers la position d'ablation peut être réalisé par une cinématique à fil de traction (14).

5. Dispositif d'ablation selon une des revendications précédentes, **caractérisé en ce que** l'applicateur d'ablation (12) est constitué par un système d'électrodes multiples dont les électrodes isolées (13) alignées les unes contre les autres dans le sens axial sont composées d'un matériau très flexible.

6. Dispositif d'ablation selon une des revendications précédentes, **caractérisé en ce que** l'applicateur d'ablation (12), dans sa position d'ablation périphérique en forme d'arc de cercle, recouvre un angle périphérique (P) d'au moins 180°.
